(19) **Europäisches Patentamt European Patent Office Office européen des brevets**

(11) **EP 2 512 434 B1**

(12) **EUROPEAN PATENT SPECIFICATION**

(45) Date of publication and mention of the grant of the patent:
**16.11.2016 Bulletin 2016/46**

(21) Application number: **10795559.3**

(22) Date of filing: **14.12.2010**

(51) Int Cl.:
*A61K 8/49* (2006.01)     *A61K 8/73* (2006.01)
*A61Q 17/04* (2006.01)

(86) International application number:
**PCT/US2010/060240**

(87) International publication number:
**WO 2011/084410 (14.07.2011 Gazette 2011/28)**

(54) **SUNSCREEN COMPOSITIONS INCORPORATING METHYLCELLULOSE AS AN SPF AND/OR PPD BOOSTER AND METHODS**

SONNENSCHUTZZUSAMMENSETZUNG MIT METHYLCELLULOSE ALS SPF UND-/ODER PPD-VERSTÄRKER UND VERFAHREN

COMPOSITIONS D'ÉCRAN SOLAIRE À BASE DE MÉTHYLCELLULOSE SERVANT DE RENFORÇATEUR DE SPF ET/OU PPD ET PROCÉDÉS ASSOCIÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.12.2009 US 287039 P**

(43) Date of publication of application:
**24.10.2012 Bulletin 2012/43**

(73) Proprietor: **Dow Global Technologies LLC Midland, MI 48674 (US)**

(72) Inventors:
• **FLETCHER, Robert Midland MI 48642 (US)**
• **GALL, James Midland MI 48642 (US)**

(74) Representative: **Beck Greener Fulwood House 12 Fulwood Place London WC1V 6HR (GB)**

(56) References cited:
**WO-A2-2010/048124     US-A1- 2003 007 944**

• **Amerchol: "SolTerra Boost technical information", , March 2009 (2009-03), XP002684058, Retrieved from the Internet: URL:http://msdssearch.dow.com/PublishedLit eratureDOWCOM/dh_0234/0901b80380234aec.p df ?filepath=suncare/pdfs/noreg/324-00321.pdf &fromPage=GetDoc [retrieved on 2012-09-24]**
• **"Boosting SPF", COSMETICS & TOILETRIES MAGAZINE, vol. 124, no. 1, January 2009 (2009-01), page 80, XP002684077,**

EP 2 512 434 B1

**Description**

**Field**

The present invention relates to sunscreen compositions.

**Background**

Many countries now require quantifiable UVA protection on sunscreen labels as well as UVB. Achieving today's UVA and UVB label claims can be challenging because high concentrations of sunscreens can be expensive. Accordingly, there is a need for sunscreen boosters which will help achieve high SPF (sun protection factor) and high UVA protection (for example, as measured by the PPD (Persistent Pigment Darkening) method) with lower active concentrations.

**Summary**

In one embodiment, the present invention provides sunscreen compositions, consisting of:

organic pigment particulates;
methylcellulose;
water; and

optionally

an additional sunscreen agent selected from octyl methoxycinnamate, avobenzone, para aminobenzoic acid, homosalate, benzophenones, benzylidenes or salicylates; and at least one of cosmetically acceptable emollients, vitamins, moisturisers, conditioners, oils, silicones, suspending agents, opacifiers/pearlisers, surfactants, emulsifiers, preservatives, rheology modifiers, colorants, pH adjustors, propellants, reducing agents, anti-oxidants, fragrances, foaming or de-foaming agents, tanning agents, insect repellants, or biocides,

wherein the organic pigment particulates are bisoctrizole.
In another embodiment, the present invention provides methods of boosting the SPF and or PPD of a sunscreen composition consisting of

organic pigment particulates,
water; and

optionally

an additional sunscreen agent selected from octyl methoxycinnamate, avobenzone, para aminobenzoic acid, homosalate, benzophenones, benzylidenes or salicylates; and at least one of cosmetically acceptable emollients, vitamins, moisturisers, conditioners, oils, silicones, suspending agents, opacifiers/pearlisers, surfactants, emulsifiers, preservatives, rheology modifiers, colorants, pH adjustors, propellants, reducing agents, anti-oxidants, fragrances, foaming or de-foaming agents, tanning agents, insect repellants, or biocides;

the method comprising including methylcellulose in the sunscreen composition, wherein the organic pigment particulates are bisoctrizole.

**Detailed Description**

The present invention provides sunscreen compositions, consisting of:

organic pigment particulates;
methylcellulose;
water; and

optionally

an additional sunscreen agent selected from octyl methoxycinnamate, avobenzone, para aminobenzoic acid, homosalate, benzophenones, benzylidenes or salicylates; and at least one of cosmetically acceptable emollients, vitamins, moisturisers, conditioners, oils, silicones, suspending agents, opacifiers/pearlisers, surfactants, emulsifiers, preservatives, rheology modifiers, colorants, pH adjustors, propellants, reducing agents, anti-oxidants, fragrances, foaming or de-foaming agents, tanning agents, insect repellants, or biocides,

wherein the organic pigment particulates are bisoctrizole.

"Organic pigment particulate" refers to carbon-based particles of less than about 250 nm, preferably less than about 200 nm.

The organic pigment particulates are bisoctrizole. Bisoctrizole (INCI name: methylene bis-benzotriazolyl tetramethylbutylphenol) is a benzotriazole based organic compound which acts as a broad spectrum UV absorber, effective in both UVA and UVB ranges. Bisoctrizole is available from Ciba Specialty Chemicals under the tradename TINOSORB M. In one embodiment, the organic pigment particulates are present in an amount from about 0.1% to about 50% by weight of the composition, and more preferably 1% to about 25%. In a most preferred embodiment, the organic pigment particulates are present in an amount constituting about 10% active.

In one embodiment, the sunscreen compositions of the present inventions further comprise an additional sunscreen agent, wherein the sunscreen agent is at least one of octyl methoxycinnamate, avobenzone, para aminobenzoic acid, homosalate, benzophenones, benzylidenes or salicylates.

Preferably, the additional sunscreen agent is octyl methoxycinnamate.

In one embodiment, the additional sunscreen agents are present in an amount from about 0.1% to about 50% by weight of the composition, and more preferably 1% to about 25%.

Methylcellulose is generally available under the tradename METHOCEL A (The Dow Chemical Company). The polymeric backbone of cellulose is a repeating structure of anhydroglucose units. Treatment of cellulosic fibers with caustic solution, followed by methyl chloride, yields cellulose ethers substituted with methoxy groups. The term "DS" refers to the degree of methoxyl substitution per anhydroglucose unit. Preferably, the methylcellulose has a $DS_{methoxyl}$ of about 1.47 to about 3.08.

In one embodiment, the methylcellulose has a viscosity at 2% concentration in water at 20°C, of about 1 cps to about 100,000 cps, preferably about 5 cps to about 30cps, most preferably about 15 cps.

In one embodiment, the methylcellulose is present in an amount from about 0.01% to about 30% by weight of the composition, preferably from about 0.05% to about 15%, and more preferably greater than 1% to about 5%.

In one embodiment, the sunscreen composition is substantially free of hydroxypropyl methylcellulose.

To make the sunscreen compositions, the organic pigment particulates are often added to the water phase.

Compositions of the present invention can further incorporate other ingredients known in the art of sunscreen formulations including at least one of cosmetically acceptable emollients, vitamins, moisturizers, conditioners, oils, silicones, suspending agents, opacifiers/pearlizers, surfactants, emulsifiers, preservatives, rheology modifiers, colorants, pH adjustors, propellants, reducing agents, anti-oxidants, fragrances, foaming or de-foaming agents, tanning agents, insect repellants, or biocides. Preferably, sunscreen compositions of the present invention include at least one of a humectant, a surfactant, an emollient, and a preservative.

In another embodiment, the present invention provides methods of boosting the SPF and/or PPD of a sunscreen composition consisting of organic pigment particulates; water; and optionally an additional sunscreen agent selected from octyl methoxycinnamate, avobenzone, para aminobenzoic acid, homosalate, benzophenones, benzylidenes or salicylates; and at least one of cosmetically acceptable emollients, vitamins, moisturisers, conditioners, oils, silicones, suspending agents, opacifiers/pearlisers, surfactants, emulsifiers, preservatives, rheology modifiers, colorants, pH adjustors, propellants, reducing agents, anti-oxidants, fragrances, foaming or de-foaming agents, tanning agents, insect repellants, or biocides, the method comprising including methylcellulose in the sunscreen composition, wherein the organic pigment particulates are bisoctrizole. In one embodiment, the sunscreen has an SPF that is at least about 25% higher, preferably at least about 50% higher, more preferably greater than about 50% higher, than sunscreens where methylcellulose is not present. In one embodiment, the sunscreen has an PPD that is at least about 25% higher, preferably at least about 50% higher, more preferably greater than about 50% higher, than sunscreens where methylcellulose is not present. In a preferred embodiment, both the SPF and the PPD are at least about 25% higher, preferably at least about 50% higher, more preferably greater than about 50% higher, than sunscreens where methylcellulose is not present.

## Examples

The following examples are for illustrative purposes only and are not intended to limit the scope of the present invention. All percentages are by weight unless otherwise specified.

## Example 1

Exemplary sunscreen compositions contain the components recited in TABLE 1.

**TABLE 1**

|   | Component | Batch 1 |
|---|---|---|
| A | Deionized Water | 77.9 |
|   | Propylene Glycol | 1.0 |
|   | GLUCAM E-10 methyl gluceth-10 | 1.0 |
|   | LIQUAPAR OPTIMA phenoxyethanol, methylparaben, isopropylparaben, isobutylparaben, and butylparaben | 0.9 |
|   | METHOCEL A methylcellulose | 2.0 |
|   | Xanthum Gum | 0.2 |
|   | Bisoctrizole (50% dispersion) | 10.0 |
| B | PEG 40 stearate | 1.0 |
|   | gylceryl stearate | 1.0 |
|   | PROMULGEN D cetearyl alcohol and ceteareth-20 | 3.0 |
|   | DC 200 Dimethicone | 2.0 |

METHOCEL A methylcellulose (DS 1.47-3.08; viscosity 10-20cps at 2%) is slowly added to about 80°C water. Once the methylcellulose is hydrated, the remaining Phase A (water) ingredients are added and the mixture maintained at 80°C for about two hours.

The Phase B (oil) ingredients are combined in a separate container, heated to, and maintained at, about 80°C for about two hours. Phase A and Phase B are then mixed and homogenized at about 11,000 rpm for about 60 seconds using a Silverson IKA Homogenizer.

## Example 2 (Comparative)

Comparative sunscreen compositions contain the components recited in TABLE 2.

**TABLE 2**

|   | Component | Comparative Batch A |
|---|---|---|
| A | Deionized Water | 79.9 |
|   | Propylene Glycol | 1.0 |
|   | GLUCAM E-10 methyl gluceth-10 | 1.0 |
|   | LIQUAPAR OPTIMA phenoxyethanol, methylparaben, isopropylparaben, isobutylparaben, and butylparaben | 0.9 |
|   | METHOCEL A methylcellulose | 0.0 |
|   | Xanthum Gum | 0.2 |
|   | Bisoctrizole (50% dispersion) | 10.0 |
| B | PEG 40 stearate | 1.0 |
|   | gylceryl stearate | 1.0 |
|   | PROMULGEN D cetearyl alcohol and ceteareth-20 | 3.0 |
|   | DC 200 Dimethicone | 2.0 |

Comparative Batch A is prepared substantially as described above with respect to Batch 1, but without methylcellulose.

## Example 3

Sunscreen compositions prepared substantially according to the protocols of Examples 1 and 2 were prepared. Their SPF values determined and recited in TABLE 3A.

**TABLE 3A**

|  | Batch 1 | Comparative Batch A |
|---|---|---|
| SPF | 20.9 ± 2.2_ | 12.5 ± _4.0_ |

The SPF was determined using an *in vitro* technique substantially according to the following protocol:

Initially, the weight of a roughened PMMA substrate (purchased from SCHÖNBERG GmbH & Co. KG, Hamburg / Germany,) is measured. The batch to be tested is then deposited on the substrate and then quickly leveled with a 7 micron draw down bar to achieve a thin, uniform layer. The layer is allowed to dry for about 20 minutes, and the weight of the substrate plus dry uniform layer is determined.

The UV absorption of dry uniform layer is measured using a LABSPHERE 1000S spectrometer at multiple points on the layer.
The percent solids of the layer is measured using a METTLER LP 16 solids analyzer. Using the weight of the dry film, and the solids content of the layer, the weight, and consequently the density of the original wet layer immediately after deposition can be calculated. Using this information, the SPF can be calculated by the following equation:

$$ SPF = \frac{\int_{290nm}^{400nm} E(\lambda)S(\lambda)\partial\lambda}{\int_{290nm}^{400nm} E(\lambda)S(\lambda)10^{(-A(\lambda))}\partial\lambda} $$

Where $E(\lambda)$ = spectral irradiance of the Standard Sun Spectrum; $S(\lambda)$ = erythemal action spectrum at wavelength $\lambda$; and $A(\lambda)$ = corrected spectral absorbance at wavelength $\lambda$ (a correction factor is calculated to extrapolate the data to establish what the absorbance would be at a wet layer density of 2.0 mg/cm$^2$ (using the original wet layer immediately after deposition).
The results in TABLE 3A are averages of at least seven measurements for each batch. The present invention results in SPF boosts of 67% as opposed to no methylcellulose.
For the PPD measurements, the SPF measurement procedure described above is used to obtain the absorption spectrum. The absorption spectrum is then analyzed using a method recommended by Colipa, the European Cosmetics Association, for measuring the PPD of sunscreens: Colipa UVA Method 2009. The PPD values determined are recited in TABLE 3B.

**TABLE 3B**

|  | Batch 1 | Comparative Batch A |
|---|---|---|
| PPD | 19.4 ± 2.0 | 11.9 ± 3.6 |

The present invention results in PPD boosts of 63% as opposed to no methylcellulose.

## Claims

1.  A sunscreen composition, consisting of:

> organic pigment particulates;
> methylcellulose
> water; and

optionally

> an additional sunscreen agent selected from octyl methoxycinnamate, avobenzone, para aminobenzoic acid, homosalate, benzophenones, benzylidenes or salicylates; and
> at least one of cosmetically acceptable emollients, vitamins, moisturisers, conditioners, oils, silicones, suspending agents, opacifiers/pearlisers, surfactants, emulsifiers, preservatives, rheology modifiers, colorants, pH adjustors, propellants, reducing agents, anti-oxidants, fragrances, foaming or de-foaming agents, tanning agents, insect repellants, or biocides

wherein the organic pigment particulates are bisoctrizole.

2. The sunscreen composition of claim 1, wherein the organic pigment particulates are present in an amount from 0.1% to 50% by weight of the composition, and more preferably 1% to 25%.

3. The sunscreen composition of claim 1, wherein the methylcellulose is present in an amount from greater than 1% to 5%.

4. The sunscreen composition of claim 1, wherein the methylcellulose has a $DS_{methoxyl}$ of 1.47 to 3.08 (the term "DS" refers to the degree of methoxyl substitution per anhydroglucose unit).

5. The sunscreen composition of claim 1, wherein the methylcellulose has a viscosity at 2% in water at 20 °C of 1cps (0.001 Pa·s) to 100,000cps (100 Pa·s), preferably 15cps (0.015 Pa·s).

6. The sunscreen composition of claim 1, wherein sunscreen composition is substantially free of hydroxypropyl methylcellulose.

7. The sunscreen composition of claim 1, comprising at least one additional sunscreen agent.

8. A method of boosting at least one of the SPF or PPD of a sunscreen composition consisting of

> organic pigment particulates,
> water; and

optionally

> an additional sunscreen agent selected from octyl methoxycinnamate, avobenzone, para aminobenzoic acid, homosalate, benzophenones, benzylidenes or salicylates; and at least one of cosmetically acceptable emollients, vitamins, moisturisers, conditioners, oils, silicones, suspending agents, opacifiers/pearlisers, surfactants, emulsifiers, preservatives, rheology modifiers, colorants, pH adjustors, propellants, reducing agents, anti-oxidants, fragrances, foaming or de-foaming agents, tanning agents, insect repellants, or biocides;

the method comprising:

> including methylcellulose in the sunscreen composition

wherein the organic pigment particulates are bisoctrizole.

9. The method of claim 8, wherein the sunscreen has an SPF that is at least 25% higher than sunscreens where methylcellulose is not present.

10. The method of claim 8 or 9, wherein the sunscreen has a PPD that is at least 25% higher than sunscreens where methylcellulose is not present.

**Patentansprüche**

1. Eine Sonnenschutzzusammensetzung, die aus Folgendem besteht:

organischen Pigmentpartikeln;
Methylcellulose
Wasser; und
optional
einem zusätzlichen Sonnenschutzmittel, ausgewählt aus Octylmethoxycinnamat, Avobenzon, para-Aminobenzoesäure, Homosalat, Benzophenonen, Benzylidenen oder
Salicylaten und mindestens einem von kosmetisch zulässigen Emoliens, Vitaminen, Feuchtigkeitscremes, Konditionierern, Ölen, Silikonen, Suspensionsmitteln,
opazitätserhöhenden Mitteln/Perlmitteln, Tensiden, Emulgatoren, Konservierungsstoffen, Rheologiewandlern, Färbemitteln, pH-Angleichungsmitteln, Treibmitteln, Reduktionsmitteln, Antioxidationsmitteln, Duftstoffen, Schaummitteln oder
Antischaummitteln, Gerbmitteln, Insektenabwehrmitteln oder Bioziden,
wobei die organischen Pigmentpartikel Bisoctrizol sind.

2. Sonnenschutzzusammensetzung gemäß Anspruch 1, wobei die organischen Pigmentpartikel in einer Menge von 0,1 Gew.-% bis 50 Gew.-% der Zusammensetzung und noch besser 1 % bis 25 % vorhanden sind.

3. Sonnenschutzzusammensetzung gemäß Anspruch 1, wobei die Methylcellulose in einer Menge von mehr als 1 % bis 5 % vorhanden ist.

4. Sonnenschutzzusammensetzung gemäß Anspruch 1, wobei die Methylcellulose einen $SG_{methoxyl}$ von 1,47 bis 3,08 aufweist (der Begriff "SG" bezieht sich auf den Grad an Methoxylsubstitution pro Anhydroglucoseeinheit).

5. Sonnenschutzzusammensetzung gemäß Anspruch 1, wobei die Methylcellulose eine Viskosität bei 2 % in Wasser bei 20 °C von 1 cps (0,001 Pa•s) bis 100,000 cps (100 Pa•s), vorzugsweise 15 cps (0,015 Pa•s), aufweist.

6. Sonnenschutzzusammensetzung gemäß Anspruch 1, wobei die Sonnenschutzzusammensetzung im Wesentlichen frei von Hydroxypropylmethylcellulose ist.

7. Sonnenschutzzusammensetzung gemäß Anspruch 1, die mindestens ein zusätzliches Sonnenschutzmittel beinhaltet.

8. Ein Verfahren zum Verstärken von mindestens einem des SPF oder PPD einer Sonnenschutzzusammensetzung, die aus Folgendem besteht:

   organischen Pigmentpartikeln;
   Wasser; und
   optional
   einem zusätzlichen Sonnenschutzmittel, ausgewählt aus Octylmethoxycinnamat, Avobenzon, para-Aminobenzoesäure, Homosalat, Benzophenonen, Benzylidenen oder
   Salicylaten und mindestens einem von kosmetisch zulässigen Emoliens, Vitaminen, Feuchtigkeitscremes, Konditionierern, Ölen, Silikonen, Suspensionsmitteln,
   opazitätserhöhenden Mitteln/Perlmitteln, Tensiden, Emulgatoren, Konservierungsstoffen, Rheologiewandlern, Färbemitteln, pH-Angleichungsmitteln, Treibmitteln, Reduktionsmitteln, Antioxidationsmitteln, Duftstoffen, Schaummitteln oder
   Antischaummitteln, Gerbmitteln, Insektenabwehrmitteln oder Bioziden;
   wobei das Verfahren Folgendes beinhaltet:

      einschließlich von Methylcellulose in der Sonnenschutzzusammensetzung wobei die organischen Pigmentpartikel Bisoctrizol sind.

9. Verfahren gemäß Anspruch 8, wobei der Sonnenschutz ein SPF aufweist, das mindestens 25 % höher als Sonnenschutz ist, bei dem keine Methylcellulose vorliegt.

10. Verfahren gemäß Anspruch 8 oder 9, wobei der Sonnenschutz ein PPD aufweist, das mindestens 25 % höher als Sonnenschutz ist, bei dem keine Methylcellulose vorliegt.

**Revendications**

1. Une composition d'écran solaire, constituée :

de matière particulaire de pigment organique ;
de méthylcellulose
d'eau ; et
facultativement
d'un agent d'écran solaire supplémentaire sélectionné parmi du méthoxycinnamate d'octyle, de l'avobenzone, de l'acide para-aminobenzoïque, de l'homosalate, des benzophénones, des benzylidènes ou des salicylates ; et
d'au moins un élément parmi des émollients acceptables d'un point de vue cosmétique,
des vitamines, des hydratants, des conditionneurs, des huiles, des silicones, des agents de mise en suspension, des opacifiants/agents nacrants, des tensioactifs, des émulsifiants, des conservateurs, des modificateurs de rhéologie, des colorants, des agents d'ajustement de pH, des gaz propulseurs, des agents réducteurs, des antioxydants, des parfums, des moussants ou anti-mousses, des agents de bronzage,
des répulsifs à insectes, ou des biocides
dans laquelle la matière particulaire de pigment organique est du bisoctrizole.

2. La composition d'écran solaire de la revendication 1, dans laquelle la matière particulaire de pigment organique est présente en une quantité allant de 0,1 % à 50 % en poids de la composition, et plus préférablement de 1 % à 25 %.

3. La composition d'écran solaire de la revendication 1, dans laquelle la méthylcellulose est présente en une quantité allant de plus de 1 % à 5 %.

4. La composition d'écran solaire de la revendication 1, dans laquelle la méthylcellulose a un $DS_{méthoxyl}$ de 1,47 à 3,08 (le terme « DS » fait référence au degré de substitution méthoxyl par unité d'anhydroglucose).

5. La composition d'écran solaire de la revendication 1, dans laquelle la méthylcellulose a une viscosité à 2 % dans de l'eau à 20 °C de 1 cps (0,001 Pa·s) à 100 000 cps (100 Pa·s), de préférence 15 cps (0,015 Pa·s).

6. La composition d'écran solaire de la revendication 1, la composition d'écran solaire étant substantiellement dépourvue d'hydroxypropyl méthylcellulose.

7. La composition d'écran solaire de la revendication 1, comprenant au moins un agent d'écran solaire supplémentaire.

8. Une méthode d'amplification d'au moins soit le FPS, soit la PPD d'une composition d'écran solaire constituée :

de matière particulaire de pigment organique,
d'eau ; et
facultativement
d'un agent d'écran solaire supplémentaire sélectionné parmi du méthoxycinnamate d'octyle, de l'avobenzone, de l'acide para-aminobenzoïque, de l'homosalate, des benzophénones, des benzylidènes ou des salicylates ; et
d'au moins un élément parmi des émollients acceptables d'un point de vue cosmétique,
des vitamines, des hydratants, des conditionneurs, des huiles, des silicones, des agents de mise en suspension, des opacifiants/agents nacrants, des tensioactifs, des émulsifiants, des conservateurs, des modificateurs de rhéologie, des colorants, des agents d'ajustement de pH, des gaz propulseurs, des agents réducteurs, des antioxydants, des parfums, des moussants ou anti-mousses, des agents de bronzage,
des répulsifs à insectes, ou des biocides ;
la méthode comprenant :

le fait d'inclure la méthylcellulose dans la composition d'écran solaire dans laquelle la matière particulaire de pigment organique est du bisoctrizole.

9. La méthode de la revendication 8, dans laquelle l'écran solaire a un FPS qui est au moins 25 % plus élevé que les écrans solaires où la méthylcellulose n'est pas présente.

10. La méthode de la revendication 8 ou de la revendication 9, dans laquelle l'écran solaire a une PPD qui est au moins 25 % plus élevée que les écrans solaires où la méthylcellulose n'est pas présente.